# EUROPEAN PATENT APPLICATION

(11) **EP 4 026 550 A1**
(43) Date of publication of application: **13.07.2022**
(21) Application number: 20859774.0
(22) Date of filing: 04.09.2020
(51) Int. Cl.: A61K 35/38, A61K 35/28, A61P 5/00, A61P 43/00, A23L 33/10

(54) **COMPOSITION FOR PREVENTING OR TREATING SALIVARY GLAND DISESAES USING CELL-DERIVED VESICLE**

(30) Priority: 06.09.2019 KR 20190110816
(71) Applicant: MDimune Inc., Seoul 04790 (KR); Inha-Industry Partnership Institute, Incheon 22212 (KR)
(72) Inventor: BAE, Shin Gyu, Seongnam-si Gyeonggi-do 13547 (KR); OH, Seung Wook, Suwon-si Gyeonggi-do 16435 (KR); KIM, Se Hee, Seoul 04728 (KR); HAN, Sung Hoon, Seoul 01835 (KR); CHOI, Jeong Seok, Incheon 21995 (KR); KIM, Jeong Mi, Seoul 04422 (KR)
(74) Representative: Boult Wade Tennant LLP
(86) International application number: PCT/KR2020/011957
(87) International publication number: WO 2021/045567

(57) **Abstract**

The present invention relates to a salivary gland therapeutic agent using the effects of a cell-derived vesicle of enhancing the proliferation capacity of salivary gland cells, promoting an amylase activity, and enhancing transepithelial resistance. A pharmaceutical composition comprising the cell-derived vesicle according to the present invention has the effects of enhancing the proliferation capacity of salivary gland cells damaged by radiation, promoting amylase activity, increasing transepithelial resistance, enhancing the expression of Aquaporin 5, and increasing the amount of saliva secretion. Therefore, the pharmaceutical composition comprising the cell-derived vesicle of the present invention can be used for preventing and treating salivary gland diseases, and thus can be widely used in the pharmaceutical industry and health functional food field.

## Description

### [Technical Field]

The present invention relates to a therapeutic agent for salivary glands using the effects of cell-derived vesicles for enhancing the proliferation of salivary gland cells, promoting amylase activity, increasing transepithelial resistance, enhancing expression of Aquaporin 5 and increasing the amount of saliva secretion.

### [Background Art]

Microvesicles are a type of cell organelle that generally has a size of 0.03 µm to 1 µm and are naturally separated from the cell membrane in almost all types of cells to have a phospholipid bilayer membrane, which is the structure of the cell membrane. These vesicles are basic cellular tools for metabolism, transport of metabolites, enzyme storage, chemical reactions, etc. They are known to play a mediating role in cell-to-cell signaling by delivering mRNAs, miRNAs, and proteins between cells.

Meanwhile, salivary glands are a generic term for exocrine glands that open in the oral cavity of higher vertebrates than reptiles, and most of them secrete mucus, but some secrete saliva as digestive juices. In mammals, there are submandibular glands, sublingual glands, and parotid glands. The submandibular and sublingual glands are also present in lower vertebrates, while the parotid gland is distinct in mammals. All three belong to branched complex acinar glands and form large adenomas. The parotid gland consists only of serous gland cells, whereas the submandibular and sublingual glands consist of serous gland cells and mucinous cells. The epithelium of both excretory ducts consists of cylindrical cells. Amylase and α-D-glucose hydrolase are secreted from salivary glands, and NaCl is secreted from the excretory duct to activate amylase. It is known that in mainly parotid glands, or to some extent submandibular glands among the salivary glands, the substances secreted in the gland migrate to the blood through specific cells to induce hormonal activity. Further, these salivary glands are called large salivary glands, and separately, small-sized salivary glands located in various places in the oral cavity that participate in salivation are distinguished as small salivary glands.

Salivary glands are important body tissues that secrete enzymes such as amylase that helps digestion, but when the salivary gland tissues are damaged, there is no effective treatment with few side effects. Therefore, there is a need for research and development for a therapeutic agent for salivary glands derived from a living body with fewer side effects.

### [Disclosure]

### [Technical Problem]

Accordingly, the present inventors have confirmed that cell-derived vesicles are effective in treating damaged salivary glands while searching and researching substances effective for treating salivary glands to complete the present invention.

Therefore, an object of the present invention is to provide a pharmaceutical composition for preventing or treating salivary gland diseases, comprising cell-derived vesicles.

### [Technical Solution]

In order to achieve the above object, the present invention provides a pharmaceutical composition for preventing or treating salivary gland disease, the composition comprising cell-derived vesicles.

Further, the present invention provides a health functional food composition for preventing or alleviating salivary gland disease, the composition comprising cell-derived vesicles.

Further, the present invention provides a method for preventing or treating salivary gland disease, the method comprising the step of treating a subject with cell-derived vesicles prepared by a manufacturing method comprising the step of extruding a sample containing cells into micropores.

Further, the present invention provides the use of cell-derived vesicles as a medicament for preventing or treating salivary gland diseases, wherein the cell-derived vesicles prepared by manufacturing method comprising the step of extruding a sample containing cells into micropores.

Further, the present invention provides the use of cell-derived vesicles prepared by manufacturing method comprising the step of extruding a sample containing cells into micropores for manufacturing a pharmaceutical composition for preventing or treating salivary gland disease.

### [Advantageous Effects]

According to the present invention, the pharmaceutical composition including the cell-derived vesicles have the effects on salivary gland cells damaged by irradiation, wherein the effects are enhancing the proliferation capacity, promoting the activity of amylase, increasing transepithelial resistance, enhancing the expression of Aquaporin 5 and increasing the amount of salivary secretion. Thus, the pharmaceutical composition of the present invention, comprising the cell-derived vesicles, is used for the prevention and treatment of salivary gland diseases so that the pharmaceutical composition can be widely used in the pharmaceutical industry and health functional food field.

### [Description of Drawings]

FIG. 1 illustrates a result of measuring changes in the proliferation capacity of parotid gland cells according to irradiation.
FIG. 2 illustrates a result of measuring changes in proliferation capacity of parotid gland cells damaged by irradiation, when treated with AdMSC-CDV.
FIG. 3 illustrates the effect of AdMSC-CDV which enhancing proliferation capacity of parotid gland cells damaged by irradiation after 9 days of irradiation.
FIG. 4 illustrates a result of measuring changes in the activity of amylase for parotid gland cells damaged by irradiation, when treated with AdMSC-CDV.
FIG. 5 illustrates a result of measuring changes in transepithelial resistance of parotid gland cells damaged by irradiation, when treated with AdMSC-CDV.
FIG. 6 illustrates a result of measuring changes in the proliferation capacity of submandibular gland cells according to irradiation.
FIG. 7 illustrates a result of measuring changes in proliferation capacity of submandibular gland cells damaged by irradiation, when treated with AdMSC-CDV.
FIG. 8 illustrates the effect of AdMSC-CDV which enhancing proliferation capacity of submandibular gland cells damaged by irradiation after 9 days of irradiation.
FIG. 9 illustrates a result of measuring changes in the activity of amylase for submandibular gland cells damaged by irradiation, when treated with AdMSC-CDV.
FIG. 10 illustrates a result of measuring changes in transepithelial resistance of submandibular gland cells damaged by irradiation, when treated with AdMSC-CDV.
FIG. 11 illustrates a result of measuring changes in the proliferation capacity of sublingual gland cells according to irradiation.
FIG. 12 illustrates a result of measuring changes in proliferation capacity of sublingual gland cells damaged by irradiation, when treated with AdMSC-CDV.
FIG. 13 illustrates the effect of AdMSC-CDV which enhancing proliferation capacity of sublingual gland cells damaged by irradiation after 9 days of irradiation.
FIG. 14 illustrates a result of measuring changes in the activity of amylase for sublingual gland cells damaged by irradiation, when treated with AdMSC-CDV.
FIG. 15 illustrates a result of measuring changes in transepithelial resistance of sublingual gland cells damaged by irradiation, when treated with AdMSC-CDV.
FIG. 16 illustrates the proliferation capacity for salivary gland cells damaged by irradiation against naturally secreted exosomes and mesenchymal stem cell-derived vesicles.
FIG. 17 illustrates the transepithelial resistance against naturally secreted exosomes and adipocyte-derived mesenchymal stem cell-derived vesicles.
FIG. 18 illustrates the transepithelial resistance against naturally secreted exosomes and umbilical cord-derived mesenchymal stem cell-derived vesicles.
FIG. 19 illustrates the effect of promoting the activity of amylase for a mouse model in which the salivary gland tissue is damaged against naturally secreted exosomes and mesenchymal stem cell-derived vesicles.
FIG. 20 illustrates a result of measuring changes in salivary secretion amount when mesenchymal stem cell-derived vesicles are applied to a mouse model.

### [Modes of the Invention]

The present invention provides a pharmaceutical composition for preventing or treating salivary gland disease, the composition comprising cell-derived vesicles prepared by a manufacturing method comprising the step of extruding a sample containing cells into micropores.

As used herein, the term "cell-derived vesicles" refers to vesicles generated from cells, and is generally a type of cell organelle. The vesicles are originated from the cell membrane in almost all types of cells, and having a form of a phospholipid bilayer membrane, which is the structure of the cell membrane.

The cell-derived vesicles of the present invention may have a micrometer size, for example, 0.03 µm to 1 µm. The vesicles of the present invention are divided into the inside and outside by a lipid bilayer consisting of the cell membrane component of the derived cells. And the vesicles are having cell membrane lipids, cell membrane proteins, nucleic acids, and cell components. Also the vesicles are smaller in size than the original cells, but not limited thereto.

In the present invention, the cell may be used without limitation, in cases that can produce the cell-derived vesicles, and may be isolated from a natural organism. In addition, the cells may be derived from any type of animal including humans and non-human mammals, or plants, and may be various types of immune cells, tumor cells, stem cells, acinar cells, myoepithelial cells, or platelets. Preferably, the stem cells may be any one or more selected from the group consisting of mesenchymal stem cells, induced pluripotent stem cells, embryonic stem cells and salivary gland stem cells. Preferably, the cells may be adipocyte-derived mesenchymal stem cells or umbilical cord-derived mesenchymal stem cells, but is not limited thereto.

The cell-derived vesicles of the present invention have the effects on salivary gland cells, wherein the effects are enhancing the proliferation capacity of, promoting the activity of amylase, increasing transepithelial resistance, enhancing the expression of Aquaporin 5 and increasing the amount of salivary secretion.

The vesicle of the present invention may be prepared from a suspension containing nucleated cells by a method selected from the group consisting of extrusion, sonication, cell lysis, homogenization, freeze-thaw, electroporation, chemical treatment, mechanical degradation, and treatment of a physical stimulus applied externally to the cell, but is not limited thereto. In the present invention, as an example, the vesicles are prepared by extruding a suspension containing nucleated cells using an extruder. The extrusion force of the extruder, applied to prepare the nucleated cell-derived vesicles of the present invention, may be 5 psi to 200 psi, 10 psi to 150 psi, or 50 psi to 100 psi.

The vesicles of the present invention may be prepared by a manufacturing method comprising the step of extruding a sample containing stem cells into micropores, and preferably, the vesicles may be prepared by a step of sequentially extruding the sample through a large-sized micropore to a small-sized micropore.

The cell-derived vesicles of the present invention may have different markers expressed in the exosomes secreted in a natural state.

The diameter of the micropores of the present invention may be 0.01 µm to 100 µm, preferably 0.1 µm to 20 µm, and more preferably 0.4 µm to 10 µm.

In the present invention, the term "salivary gland disease" may be a condition in which normal function is difficult due to damage or inflammation of the salivary gland tissue, and preferably the disease may be any one selected from the group consisting of irradiation and radioactive isotope damage to the salivary gland, dry mouth, infectious acute sialadenitis, infectious chronic sialadenitis, tuberculosis, Sjogren's syndrome, sialolithiasis, sialadenosis, salivary gland tumor, and salivary gland dysfunction due to aging. More preferably, the disease may be irradiation and radioactive isotope damage to the salivary gland or dry mouth. Further, the radioactive isotope may be, for example, radioactive iodine. In particular, the main salivary glands, such as the submandibular glands and the parotid glands, are responsible for the secretion of saliva. The submandibular glands are responsible for the secretion of saliva in daily life, and the parotid glands are responsible for secretion of saliva by stimulation. However, exposure of the salivary glands to radiation for reasons such as radiation and radioactive isotope therapy causes irreversible damage to the salivary epithelial cells. This causes the tight junction between cells to loosen, thereby increasing the epithelial permeability of the salivary gland cells. When the epithelial permeability of salivary gland cells is increased, the amount of saliva secretion and saliva components may be changed by the absorption of macromolecules or bacteria which should not be absorbed, or by the abnormally operating of the secretion of amylase which a salivary enzyme, and the transport of water, ions, and the like. The cell-derived vesicles of the present invention may have the effect of enhancing cell proliferation capacity of salivary gland, promoting the activity of amylase, increasing transepithelial resistance and increasing the amount of salivary secretion. Based on the forementioned effect, the cell-derived vesicles may have the effect of improving or treating symptoms caused by the salivary gland disease.

In the present invention, the salivary glands may be parotid glands, submandibular glands or sublingual glands.

In the present invention, the cell-derived vesicles may be included in any amount (effective amount) depending on the formulation and purpose of mixture, as in cases that can exhibit a preventive or therapeutic effect on salivary gland disease. Herein, the term "effective amount" refers to the amount of active ingredient included in the composition of the present invention. In cases, the composition of the present invention, comprising the effective amount of active ingredient, may be administered to the subject to be applied, wherein the subject is a mammal or preferably a human, during the administration period suggested by a medical expert, etc. and the composition may cause intended functional and pharmacological effects such as the effect of enhancing proliferation capacity of salivary gland cell. Such the effective amount can be experimentally determined within the ordinary capacity of those skilled in the art, and it may be included preferably at a concentration of 1 × 10⁵ to 1 × 10¹³ particles/ml, more preferably 5 × 10⁷ to 1 × 10¹³ particles/ml, even more preferably 5 × 10⁸ to 1 × 10¹³ particles/ml, and still more preferably 1 × 10⁹ to 1 × 10¹³ particles/ml.

The pharmaceutical composition for preventing or treating salivary gland disease of the present invention, in addition to the active ingredient, may further include any compound or natural extract that has already been verified for safety and is known to have a preventive or therapeutic effect for salivary gland disease for the purpose of increasing/reinforcing the prevention or treatment effect of salivary gland disease.

The pharmaceutical composition of the present invention may be formulated and used in the form of oral dosage forms such as powders, granules, tablets, capsules, suspensions, emulsions, syrups, and aerosols, external preparations, suppositories, and sterile injection solutions, respectively, according to conventional methods. Carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil. The formulations are prepared using diluents or excipients (e.g., fillers, extenders, binders, humectants, disintegrants, surfactants, etc.) that are commonly used. Solid formulations for oral administration may include tablets, pills, powders, granules, capsules, etc. These solid formulations may be prepared by mixing the pharmaceutical composition of the present invention with at least one excipient (e.g., starch, calcium carbonate, sucrose, lactose, gelatin, etc.). In addition to simple excipients, lubricants (e.g., magnesium stearate and talc) may also be used. Liquid formulations for oral administration may contain suspensions, solutions for internal use, emulsions, syrups, etc. In addition to the simple diluents commonly used (e.g., water and liquid paraffin), various other excipients (e.g., humectants, sweeteners, fragrances, preservatives, etc.) may also be used. Formulations for parenteral administration may contain sterile aqueous solutions, non-aqueous solvents, suspensions, emulsions, lyophilized preparations, and suppositories. As the non-aqueous solvents and the suspensions, propylene glycol, polyethylene glycol, vegetable oil (e.g., olive oil), an injectable ester (e.g., ethyloleate), etc. may be used. As a base for the suppositories, Witepsol, Macrogol, Tween 61, cacao butter, laurin butter, glycerogelatin, etc. may be used.

The dosage of the pharmaceutical composition of the present invention varies depending on the factors, such as, the age, sex, and weight of the subject to be treated, the specific disease or pathological condition to be treated, the severity of the disease or pathological condition, the route of administration, and the determination of the prescriber. Dosage determination based on these factors is within the level of one of ordinary skill in the art, and generally, the dosage has a range from 0.01 mg/kg/day to approximately 2000 mg/kg/day. A more preferred dosage has a range from 1 mg/kg/day to 500 mg/kg/day. The pharmaceutical composition may be administered once a day or may be administered in several divided doses. The above dosages do not limit the scope of the present invention in any way.

The pharmaceutical composition of the present invention may be administered to mammals such as mice, livestock, and humans with various routes. All of the methods of administration can be expected, for example, The pharmaceutical composition may be administered by oral, rectal or intravenous, intramuscular, subcutaneous, gargling, oral salivary gland injection, intrauterine intrathecal or intracerebrovascular injection. The composition of the present invention has almost no toxicity and side effects. And thus, the composition is a medicament which can be safely used and used for a long period of time for prevention purposes.

Further, the present invention provides a health functional food composition for preventing or alleviating salivary gland disease, the composition including cell-derived vesicles prepared by a manufacturing method comprising the step of extruding a sample containing cells into micropores.

The health functional food of the present invention includes ingredients commonly added during food production, for example, proteins, carbohydrates, fats, nutrients and seasonings. For example, when manufactured as a drink, a flavoring agent or natural carbohydrate may be included as an additional ingredient in addition to the cell-derived vesicle as an active ingredient. For example, natural carbohydrates may include monosaccharides (e.g., glucose, fructose, etc.), disaccharides (e.g., maltose, sucrose, etc.), oligosaccharides, polysaccharides (e.g., dextrin, cyclodextrin, etc.), and sugar alcohols (e.g., xylitol, sorbitol, erythritol, etc.). As flavoring agents, natural flavoring agents (e.g., taumarin, stevia extract, etc.) and synthetic flavoring agents (e.g., saccharin, aspartame, etc.) may be included.

The health functional food of the present invention includes the form of tablets, capsules, pills, or liquids, and the food to which the cell-derived vesicle of the present invention can be added includes, for example, various drinks, meat, sausage, bread, candy, snacks, noodles, ice cream, dairy products, soups, ionic drinks, beverages, alcoholic beverages, gum, tea and vitamin complex.

Since the functional health food for preventing or alleviating salivary gland disease of the present invention has the same active ingredient and effect as the pharmaceutical composition for preventing or treating salivary gland disease, the description of common content between the two is excluded to avoid excessive complexity of the present specification.

Further, the present invention provides a method for preventing or treating salivary gland disease, the method comprising the step of treating a subject with the cell-derived vesicles prepared by a manufacturing method comprising the step of extruding a sample containing cells into micropores.

Further, the present invention provides a use of cell-derived vesicles as a medicament for preventing or treating salivary gland disease, wherein the cell-derived vesicles prepared by manufacturing method comprising the step of extruding a sample containing cells into micropores.

Further, the present invention provides a use of a cell-derived vesicles prepared by manufacturing method comprising the step of extruding a sample containing cells into micropores for manufacturing a pharmaceutical composition for preventing or treating salivary gland disease.

The present invention is described in detail with reference to the following examples. These examples are only for illustrative purposes and do not limit the scope of the present invention.

### Example 1. Culture of human salivary gland cells

Parotid, submandibular, and sublingual glands, which are normal tissues of the salivary glands of a patient who underwent parotid resection, were obtained and the surrounding tissues were removed under a microscope. Then, they were immediately placed in HBSS containing 1% antibiotics for primary culture and stored at 4°C until the experiment. The human-derived salivary gland tissue used in this study was approved by the Institutional Review Board of Inha University Hospital and used for the experiment (IRB approval number: NON2017-002). Fresh tissue was cut into small pieces using surgical scissors, and they were added with 0.25% collagenase Type B (Sigma Aldrich, USA) and treated at 37°C for 30 minutes. The obtained cell suspension was filtered through a 70 um strainer and centrifuged at 1500 rpm for 5 minutes. The obtained cells were suspended in DMEM/F12 (Invitrogen, USA) containing 10% FBS (ATCC, USA) and 1% antibiotics (Invitrogen, USA). After suspension, the cells were transferred to a cell culture dish and cultured in an incubator at 37°C and 5% CO₂.

### Example 2. Preparation of salivary gland tissue damaged by irradiation (IR)

Parotid epithelial cells (hPECs, hereinafter, parotid gland cells ), sublingual epithelial cells (hLECs, hereinafter, sublingual gland cells), and submandibular epithelial cells (hMECs, hereinafter, submandibular gland cells), which were subcultured to passages 3 to 6 prepared in Example 1, were irradiated with radiation using a linear accelerator 4 MV X-ray (Mevatron MD, Siemens Medical Laboratory, USA). Because the sensitivity to radiation varies depending on the cell, the parotid epithelial cells and sublingual epithelial cells were irradiated with 0, 2, and 5 Gy, respectively, and the submandibular epithelial cells were irradiated with 0, 7, and 15 Gy. They were cultured in an incubator at 37°C and 5% CO₂.

### Example 3. Preparation of adipose-derived mesenchymal stem cell-derived vesicle (AdMSC CDV)

Vesicles were prepared from adipose-derived mesenchymal stem cells through extrusion. Mesenchymal stem cells cultured in stem cell complete growth medium are washed with phosphate-buffered saline (PBS), and the washed stem cells are resuspended in PBS at a concentration of 0.25 to 1 × 10⁶ cells/ml. The suspension solution was passed through a membrane filter having a micropore size of 10 µm with an extruder, passed through a membrane filter having a micropore size of 5 µm and then passed through a membrane filter having a micropore size of 1 to 0.4 µm. The suspension obtained through this process was added with benzonase nuclease to remove DNA, and they were reacted at 37°C for 90 minutes. For purification of the DNA-removed suspension, it was passed through a membrane column by TFF (Tangential Flow Filtration). If the concentration was required, the resulting suspension was subjected to ultracentrifuge, and the precipitated layer was resuspended in phosphate-buffered saline (PBS). The resultant was filtered through a 0.2 um membrane filter to obtain mesenchymal stem cell-derived vesicles.

### Example 4. Confirmation of effect of adipose-derived mesenchymal stem cell-derived vesicles on parotid gland tissue damaged by irradiation

### 4.1 Changes in proliferation capacity upon irradiation exposure to parotid gland cells

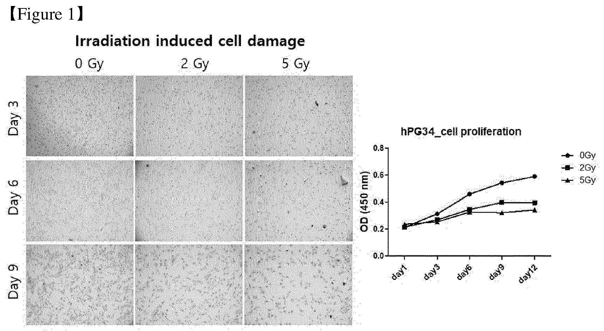

An experiment was conducted to determine how the proliferation capacity of parotid gland cells was changed when the parotid gland cells were exposed to irradiation. Specifically, parotid gland cells prepared in Example 1 were irradiated with radiation in the same manner as in Example 2 to damage the cells. Thereafter, the change in proliferation over time after irradiation was measured using CCK8 uptake assay (Dojindo, Japan). The CCK8 uptake assay is as follows. Parotid gland cells damaged by radiation were inoculated into 96 wells at 1 × 10³ cells/well, respectively. Then, they were cultured for 1, 3, 6, 9, and 12 days in a 37°C, 5% CO₂ incubator. They were added with 10 uL CCK8 reagent and cultured for 3 hours in a 37°C, 5% CO₂ incubator. The proliferation capacity was measured by measuring the absorbance at 490 nm using a 96 well plate reader (Dynex Revelation, Dynex Ltd, UK). The measured proliferation capacity is shown in FIG. 1.

As shown in FIG. 1, the degree of proliferation of parotid gland cells after 0 Gy, 2 Gy, and 5 Gy irradiation, respectively, was observed for 1, 3, 6, 9, and 12 days. As a result, it was confirmed that cell proliferation occurred well over time in the non-irradiated group (0 Gy) whereas cell proliferation was significantly reduced from the 3rd day in the group irradiated with 2 Gy or 5 Gy radiation compared to the non-irradiated group.

### 4.2 Confirmation of effect of enhancing the proliferation capacity of adipose-derived mesenchymal stem cell-derived vesicles on parotid gland tissue damaged by irradiation

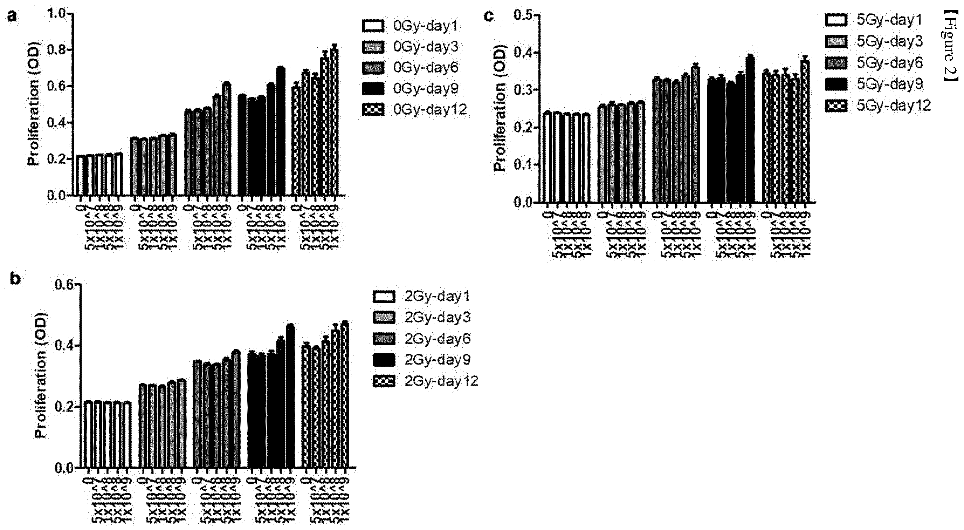

An experiment was conducted to confirm the effect of enhancing the proliferation capacity of adipose-derived mesenchymal stem cell-derived vesicles on parotid gland tissue damaged by radiation. The experimental group was set up as follows. They were divided into a non-irradiated group, a group irradiated with 2 Gy radiation, and a group irradiated with 5 Gy radiation. Subgroups with different treatment concentrations were established in which AdMSC-CDV prepared in Example 3 at 0, 5 × 10⁷, 1 × 10⁸, 5 × 10⁸ or 1 × 10⁹ particles/ml were treated in each group. The proliferation capacity of each subgroup was measured 1, 3, 6, 9 and 12 days after irradiation. Cell proliferation capacity was measured using the same method as in Example 4.1, and the measurement results are shown in FIGS. 2A to 2C.

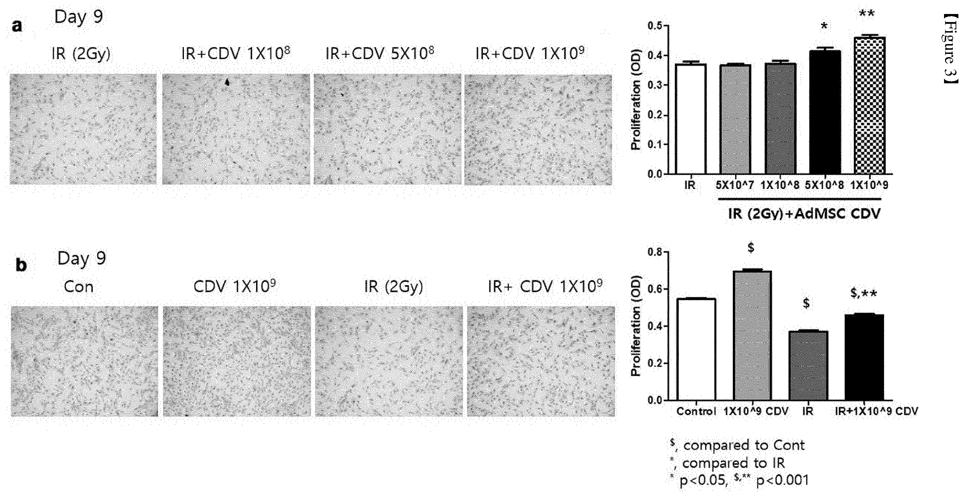

In addition, the proliferation capacity measured 9 days after irradiation damage was classified into the normal control group (no treatment), IR group (2 Gy of irradiation (IR) treatment only), AdMSC-CDV group (no irradiation treatment and 1 × 10⁹ particles/ml of AdMSC-CDV treatment) and IR + AdMSC-CDV group (2 Gy of IR treatment and 1 × 10⁸, 5 × 10⁸ or 1 × 10⁹ particles/ml of AdMSC-CDV treatment) and the comparison results are shown in FIGS. 3A and 3B.

As shown in FIG. 2A, after treatment with AdMSC-CDV at a concentration of 5 × 10⁷ to 1 × 10⁹/ml to parotid gland cells irradiated with 0 Gy of radiation, cell proliferation capacity was confirmed on 1, 3, 6, 9, and 12th day. As a result, it was confirmed that the cell proliferation capacity was increased in the groups treated with 5 × 10⁸ and 1 × 10⁹/ml concentration from 6th day. Further, it was confirmed that this effect persisted until the 12th day.

As shown in FIG. 2B, after treatment with AdMSC-CDV at a concentration of 5 × 10⁷ to 1 × 10⁹/ml to parotid gland cells irradiated with 2 Gy of radiation, cell proliferation capacity was confirmed on days 1, 3, 6, 9, and 12th day. As a result, it was confirmed that the cell proliferation capacity was increased in the groups treated with 5 × 10⁸ and 1 × 10⁹/ml concentration of AdMSC-CDV from 6th day compared to the group not treated with AdMSC-CDV. Further, it was confirmed that this effect persisted until the 12th day.

As shown in FIG. 2C, after treatment with AdMSC-CDV at a concentration of 5 × 10⁷ to 1 × 10⁹/ml to parotid gland cells irradiated with 5 Gy of radiation, cell proliferation capacity was confirmed on 1, 3, 6, 9, and 12th day. As a result, it was confirmed that the cell proliferation capacity was increased in the group treated with 1 × 10⁹/ml concentration of AdMSC-CDV compared to the group untreated with AdMSC-CDV. Further, it was confirmed that this effect persisted from 6th day to the 12th day after irradiation.

As shown in FIG 3A, the parotid gland cells irradiated with 2 Gy radiation were treated with AdMSC-CDV by concentration, and the cell proliferation capacity after 9 days was confirmed. It was confirmed that the cell proliferation capacity of the group irradiated with 2 Gy of radiation and treated with AdMSC-CDV at a concentration of 5 × 10⁸/ml or 1 × 10⁹/ml was increased in a concentration-dependent manner compared to the group irradiated with only 2 Gy of radiation.

As shown in FIG. 3B, it was confirmed that the cell proliferation capacity of the group treated with AdMSC-CDV at a concentration of 1 × 10⁹/ml without irradiation was much higher than that of the untreated group, and the cell proliferation capacity of the group irradiated with only 2 Gy of radiation was decreased compared to the untreated group, but the cell proliferation capacity of the group irradiated with 2 Gy of radiation and treated with AdMSC-CDV at a concentration of 1 × 10⁹/ml was much higher than that of the group irradiated with only 2 Gy of radiation.

Combining the above data, it was confirmed that AdMSC-CDV enhances the proliferation capacity of parotid gland cells, and the proliferation capacity of parotid gland cells damaged by radiation is also increased by AdMSC-CDV.

### 4.3 Confirmation of effect of promoting amylase activity of adipose-derived mesenchymal stem cell-derived vesicles on parotid gland tissue damaged by irradiation

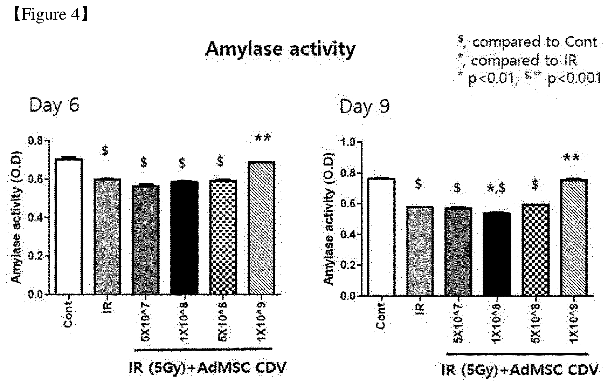

An experiment was conducted to confirm the amylase activity promoting effect of adipose-derived mesenchymal stem cell-derived vesicles on parotid gland tissue damaged by irradiation. Specifically, parotid gland cells prepared in Example 1 were irradiated in the same manner as in Example 2. The group in which parotid gland cells were not treated and the group irradiated with only 5 Gy radiation were set as controls. The group in which parotid gland cells were irradiated with 5 Gy radiation and treated with AdMSC-CDV prepared in Example 3 at a concentration of 5 × 10⁷ to 1×10⁹/ml was set as the experimental group. Specifically, the experiment was conducted in the following way. Parotid gland cells of the experimental group and the control group were inoculated into a 96-well plate at 1 × 10³ cells/well, and the cells were treated with AdMSC-CDV immediately after irradiation as set, respectively. To measure the activity of amylase secreted into the cell culture on the 6th and 9th days after irradiation, the a-amylase assay kit (Abcam, USA) was used according to the protocol, and the absorbance values were measured at 405 nm using a 96 well plate reader. The measured results are shown in FIG. 4.

As shown in FIG. 4, it was confirmed that the activity of amylase was reduced in the group irradiated with only 5 Gy radiation compared to the untreated group. The group irradiated with 5 Gy radiation and treated with AdMSC-CDV by concentration were cultured for 6 days and 9 days. It was confirmed that the amylase activity of the group irradiated with 5G y radiation and treated with 1 × 10⁹/ml AdMSC-CDV on both the 6th and 9th days was restored to a level similar to the amylase activity of the untreated group. That is, it was confirmed that AdMSC-CDV promotes the amylase activity of parotid gland cells and that the amylase activity of parotid gland cells damaged by radiation is also promoted by AdMSC-CDV.

### 4.4 Confirmation of effect of enhancing transepithelial resistance (TEER) of adipose-derived mesenchymal stem cell-derived vesicles on parotid gland tissue damaged by irradiation

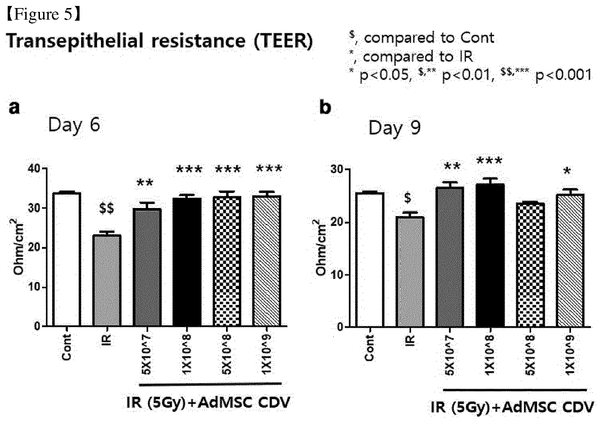

An experiment was conducted to confirm the transepithelial resistance enhancing effect of adipose-derived mesenchymal stem cell-derived vesicles on parotid gland tissue damaged by irradiation. Specifically, parotid gland cells prepared in Example 1 were irradiated in the same manner as in Example 2. The group in which parotid gland cells were not treated and the group irradiated with only 5 Gy radiation were set as controls. The group in which parotid gland cells were irradiated with 5 Gy radiation and treated with AdMSC-CDV prepared in Example 3 at a concentration of 5 × 10⁷ to 1 × 10⁹/ml was set as the experimental group. Specifically, the experiment was conducted in the following way. Parotid gland cells of the experimental group and the control group were inoculated into transwells (12 mm diameter, 0.4 um pore size, Corning, USA) at 1 × 10⁵ cells/well, and the cells were treated with AdMSC-CDV immediately after irradiation as set, respectively. Transepithelial resistance (TEER) was measured using Millicell ERS-2 (EMD Millipore Corp. USA) on the 6th and 9th days after culturing the treated cells in an incubator at 37°C and 5% CO₂. The results measured on the 6th day of irradiation are shown in FIG. 5A and the results measured on the 9th day of irradiation are shown in FIG. 5B.

As shown in FIG. 5A, transepithelial resistance (TEER) was measured 6 days after the parotid gland cells were irradiated with 5 Gy radiation. It was confirmed that the transepithelial resistance (TEER) of the group irradiated with only radiation was significantly reduced compared to the untreated group, and the transepithelial resistance (TEER) of all groups irradiated with 5 Gy radiation and then treated with AdMSC-CDV was significantly higher compared to that of the group irradiated with only radiation.

As shown in FIG. 5B, the transepithelial resistance (TEER) was measured 9 days after 5 Gy irradiation, and the results indicated that the transepithelial resistance (TEER) of the group irradiated with only radiation was reduced compared to the untreated group, and the reduced transepithelial resistance (TEER) was increased again in the group treated with 5 × 10⁷, 1 × 10⁸ and 1 × 10⁹/ml of AdMSC-CDV.

That is, it was confirmed that AdMSC-CDV increases the transepithelial resistance (TEER) of parotid gland cells and the transepithelial resistance (TEER) of parotid gland cells damaged by irradiation is also restored by AdMSC-CDV.

### Example 5. Confirmation of effect of adipose-derived mesenchymal stem cell-derived vesicles on submandibular gland tissue damaged by radiation

### 5.1 changes in proliferation capacity upon irradiation of submandibular gland cells

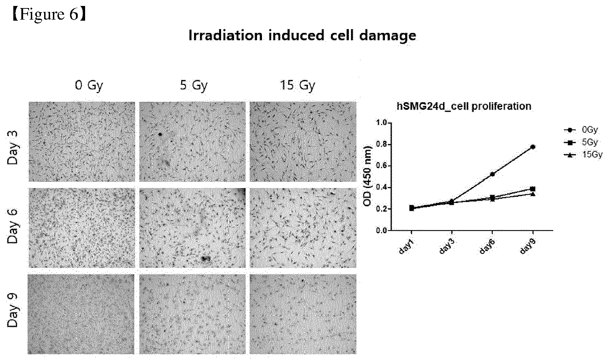

An experiment was conducted to determine how the proliferation capacity of submandibular gland cells was changed when the submandibular gland cells were irradiated with radiation. Specifically, submandibular gland cells prepared in Example 1 were irradiated with radiation in the same manner as in Example 2 to damage the cells. Thereafter, the proliferation capacity of submandibular gland cells was measured in the same manner as in Example 4.1 on 1, 3, 6, and 9th day after irradiation. The measured proliferation capacity is shown in FIG. 6.

As shown in FIG. 6, the degree of proliferation of submandibular gland cells after 0 Gy, 5 Gy, and 15 Gy irradiation, respectively, was observed for 1, 3, 6, and 9 days. As a result, it was confirmed that cell proliferation occurred well over time in the non-irradiated group (0 Gy), whereas cell proliferation was significantly reduced from the 6th day in the group irradiated with 5Gy or 15 Gy radiation compared to the non-irradiated group.

### 5.2 Confirmation of effect of enhancing proliferation capacity of adipose-derived mesenchymal stem cell-derived vesicles on submandibular gland tissue damaged by irradiation

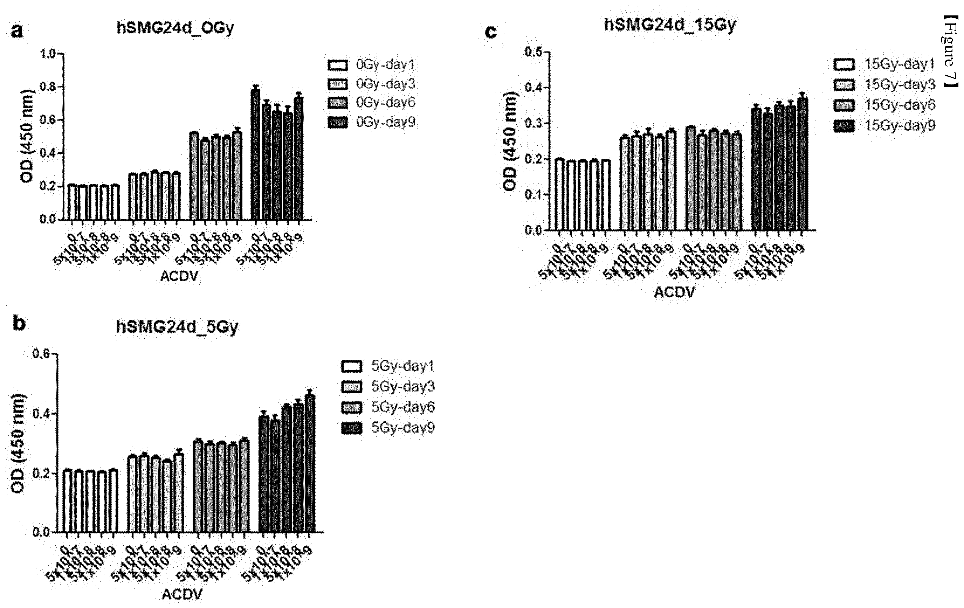

An experiment was conducted to confirm the effect of enhancing the proliferation capacity of adipose-derived mesenchymal stem cell-derived vesicles on submandibular gland tissue damaged by irradiation. The experimental group was set up as follows. They were divided into a non-irradiated group, a group irradiated with 5 Gy radiation, and a group irradiated with 15 Gy radiation. Subgroups with different treatment concentrations were established in which AdMSC-CDV prepared in Example 3 at 0, 5 × 10⁷, 1 × 10⁸, 5 × 10⁸ or 1 × 10⁹ particles/ml were treated in each group. The proliferation capacity of each subgroup was measured on 1, 3, 6, and 9the day after irradiation. Cell proliferation capacity was measured using the same method as in Example 4.1, and the measurement results are shown in FIGS. 7A to 7C.

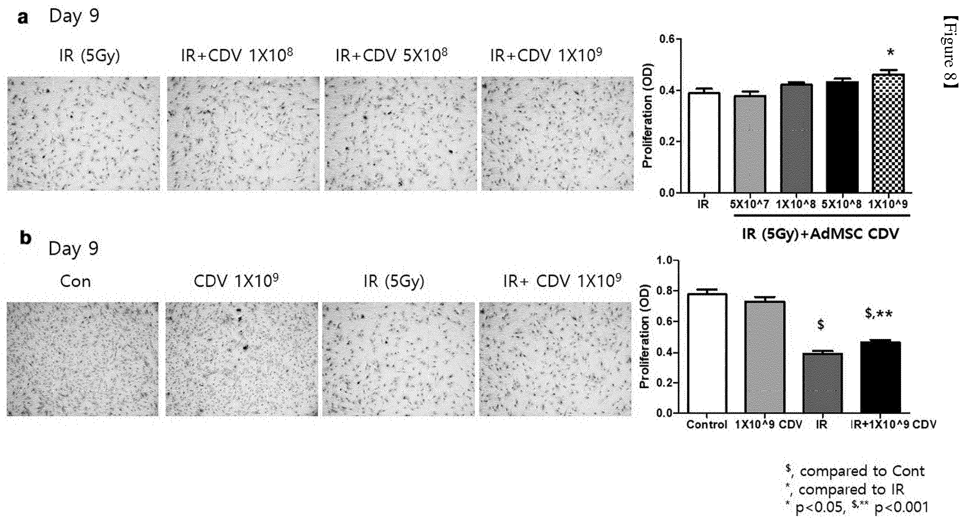

In addition, the proliferation capacity measured 9 days after irradiation damage was classified into the normal control group (no irradiation treatment and no AdMSC-CDV treatment), IR group (no AdMSC-CDV treatment and 5 Gy of irradiation (IR) treatment only), AdMSC-CDV group (no irradiation treatment and 1 × 10⁹ particles/ml of AdMSC-CDV treatment) and IR + AdMSC-CDV group (5 Gy of IR treatment and 1 × 10⁸, 5 × 10⁸ or 1 × 10⁹ particles/ml of AdMSC-CDV treatment), and the comparison results are shown in FIGS. 8A and 8B.

As shown in FIG. 7A, after treatment with AdMSC-CDV at a concentration of 5 × 10⁷ to 1 × 10⁹/ml to submandibular gland cells irradiated with 0 Gy of radiation, cell proliferation capacity was confirmed on 1, 3, 6, and 9th day. As a result, it was confirmed that there is no effect of increasing the proliferation capacity according to the AdMSC-CDV treatment.

As shown in FIG. 7B, after treatment with AdMSC-CDV at a concentration of 5 × 10⁷ to 1 × 10⁹/ml to submandibular gland cells irradiated with 5 Gy of radiation, cell proliferation capacity was confirmed on days 1, 3, 6, and 9th day. As a result, it was confirmed that the cell proliferation capacity was increased in the groups treated with 1 × 10⁸, 5 × 10⁸ and 1 × 10⁹/ml concentration of AdMSC-CDV from 9th day compared to the group untreated with AdMSC-CDV.

As shown in FIG. 7C, after treatment with AdMSC-CDV at a concentration of 5 × 10⁷ to 1 × 10⁹/ml to submandibular gland cells irradiated with 15 Gy of radiation, cell proliferation capacity was confirmed on days 1, 3, 6 and 9th day. As a result, it was confirmed that the cell proliferation capacity was increased in the group treated with 1 × 10⁹/ml concentration of AdMSC-CDV from 9th day compared to the group untreated with AdMSC-CDV.

As shown in FIG 8A, the submandibular gland cells irradiated with 5 Gy radiation were treated with AdMSC-CDV by concentration, and the cell proliferation capacity after 9 days was confirmed. It was confirmed that the cell proliferation capacity of the group irradiated with 5 Gy of radiation and treated with AdMSC-CDV at a concentration of 1 × 10⁹/ml was increased compared to the group irradiated with only 5 Gy of radiation.

As shown in FIG. 8B, it was confirmed that there was no difference in the cell proliferation capacity of the group treated with AdMSC-CDV at a concentration of 1 × 10⁹/ml without irradiation compared to that of the untreated group, and the cell proliferation capacity of the group irradiated with only 5 Gy of radiation was decreased compared to that of the untreated group, but the cell proliferation capacity of the group irradiated with 5 Gy of radiation and treated with AdMSC-CDV at a concentration of 1 × 10⁹/ml was much higher than that of the group irradiated with only 5 Gy of radiation.

Combining the above data, it was confirmed that AdMSC-CDV enhances the proliferation capacity of submandibular gland cells, and the proliferation capacity of submandibular gland cells damaged by radiation is also increased by AdMSC-CDV.

### 5.3 Confirmation of effect of promoting amylase activity of adipose-derived mesenchymal stem cell-derived vesicles on submandibular gland tissue damaged by irradiation

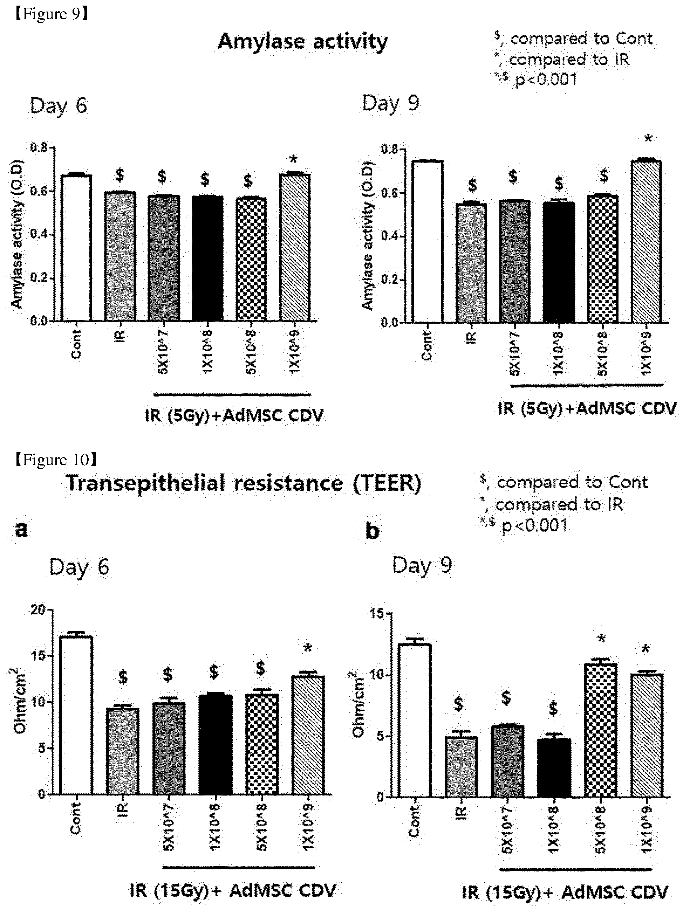

An experiment was conducted to confirm the amylase activity promoting effect of adipose-derived mesenchymal stem cell-derived vesicles on submandibular gland tissue damaged by irradiation. Specifically, submandibular gland cells prepared in Example 1 were irradiated in the same manner as in Example 2. The group in which submandibular gland cells were not treated and the group irradiated with only 5 Gy radiation were set as controls. The group in which submandibular gland cells were irradiated with 5 Gy radiation and treated with AdMSC-CDV prepared in Example 3 at a concentration of 5 × 10⁷ to 1 × 10⁹/ml was set as the experimental group. Specifically, the amylase activity was measured in the same method as in Example 4.3. and the measurement results are shown in FIG. 9.

As shown in FIG. 9, it was confirmed that the amylase activity of the group irradiated with only 5 Gy radiation was reduced compared to the untreated group. The group irradiated with 5 Gy radiation and treated with AdMSC-CDV by concentration were cultured for 6 days and 9 days. It was confirmed that the amylase activity of the group irradiated with 5 Gy radiation and treated with 1 × 10⁹/ml AdMSC-CDV on both 6th and 9th days was restored to a level similar to the amylase activity of the untreated group. That is, it was confirmed that AdMSC-CDV promotes the amylase activity of submandibular gland cells, and that the amylase activity of submandibular gland cells damaged by radiation is also restored by AdMSC-CDV.

### 5.4 Confirmation of effect of enhancing transepithelial resistance (TEER) of adipose-derived mesenchymal stem cell-derived vesicles on submandibular gland tissue damaged by irradiation

An experiment was conducted to confirm the transepithelial resistance enhancing effect of adipose-derived mesenchymal stem cell-derived vesicles on submandibular gland tissue damaged by irradiation. Specifically, submandibular gland cells prepared in Example 1 were irradiated in the same manner as in Example 2. The group in which submandibular gland cells were not treated and the group irradiated with only 15 Gy radiation were set as controls. The group in which submandibular gland cells were irradiated with 15 Gy radiation and treated with AdMSC-CDV prepared in Example 3 at a concentration of 5 × 10⁷ to 1 × 10⁹/ml was set as the experimental group. Transepithelial resistance was measured in the same manner as in Example 4.4, and the measurement results are shown in FIGS. 10A and 10B.

As shown in FIG. 10A, transepithelial resistance (TEER) was measured 6 days after the submandibular gland cells were irradiated. It was confirmed that the transepithelial resistance (TEER) of all groups irradiated with radiation was significantly reduced compared to the untreated group, and the transepithelial resistance (TEER) of groups irradiated with 15 Gy radiation and then treated with 1 × 10⁹/ml AdMSC-CDV was higher compared to those of the groups irradiated with 15 Gy radiation and then treated with 5 × 10⁷ to 5 × 10⁸/ml AdMSC-CDV.

As shown in FIG. 10B, the transepithelial resistance (TEER) was measured 9 days after irradiation, and the results indicated that the transepithelial resistance (TEER) of all groups irradiated with radiation was reduced compared to the untreated group, and the reduced transepithelial resistance (TEER) was increased again in the group treated with 5 × 10⁸ and 1 × 10⁹/ml AdMSC-CDV.

That is, it was confirmed that AdMSC-CDV increases the transepithelial resistance (TEER) of submandibular gland cells, and the transepithelial resistance (TEER) of submandibular gland cells damaged by irradiation is also restored by AdMSC-CDV.

### Example 6. Confirmation of effect of adipose-derived mesenchymal stem cell-derived vesicles on sublingual gland tissue damaged by radiation

### 6.1 Changes in proliferation capacity upon irradiation of sublingual gland cells

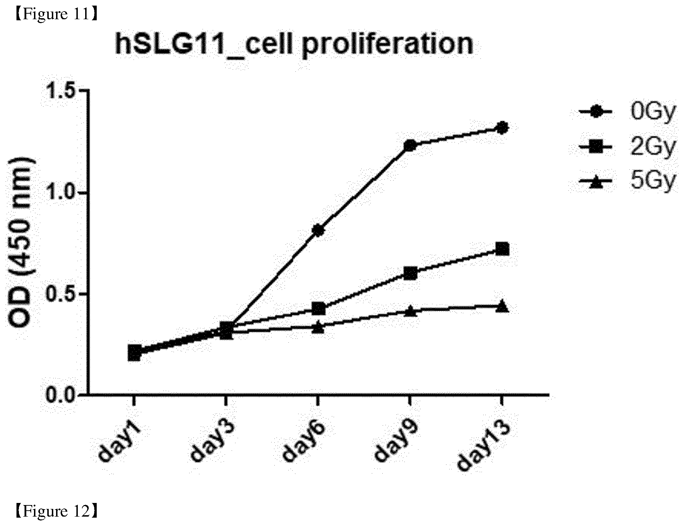

An experiment was conducted to determine how the proliferation capacity of sublingual gland cells was changed when the sublingual gland cells were irradiated with radiation. Specifically, sublingual gland cells prepared in Example 1 were irradiated with radiation in the same manner as in Example 2 to damage the cells. Thereafter, the proliferation capacity of sublingual gland cells was measured in the same manner as in Example 4.1 on 1, 3, 6, 9 and 13th days after irradiation. The measured proliferation capacity is shown in FIG. 11.

As shown in FIG. 11, the degree of proliferation of sublingual gland cells after 0 Gy, 2 Gy, and 5 Gy irradiation, respectively, was observed for 1, 3, 6, 9 and 13 days. As a result, it was confirmed that cell proliferation occurred well over time in the non-irradiated group (0 Gy), whereas cell proliferation was significantly reduced from the 6th day in the group irradiated with 2 Gy or 5 Gy radiation compared to the non-irradiated group.

### 6.2 Confirmation of effect of enhancing proliferation capacity of adipose-derived mesenchymal stem cell-derived vesicles on sublingual gland tissue damaged by irradiation

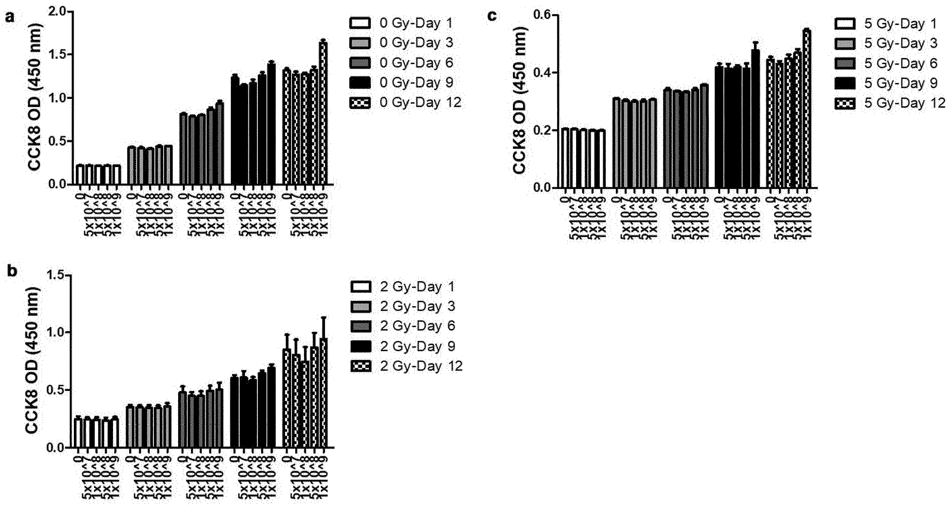

An experiment was conducted to confirm the effect of enhancing the proliferation capacity of adipose-derived mesenchymal stem cell-derived vesicles on sublingual gland tissue damaged by irradiation. The experimental group was set up as follows. They were divided into a non-irradiated group, a group irradiated with 2 Gy radiation, and a group irradiated with 5 Gy radiation. Subgroups with different treatment concentrations were established in which AdMSC-CDV prepared in Example 3 at 0, 5 × 10⁷, 1 × 10⁸, 5 × 10⁸ or 1 × 10⁹ particles/ml were treated in each group. The proliferation capacity of each subgroup was measured 1, 3, 6, 9 and 12 days after irradiation. Cell proliferation capacity was measured using the same method as in Example 4.1, and the measurement results are shown in FIGS. 12A to 12C.

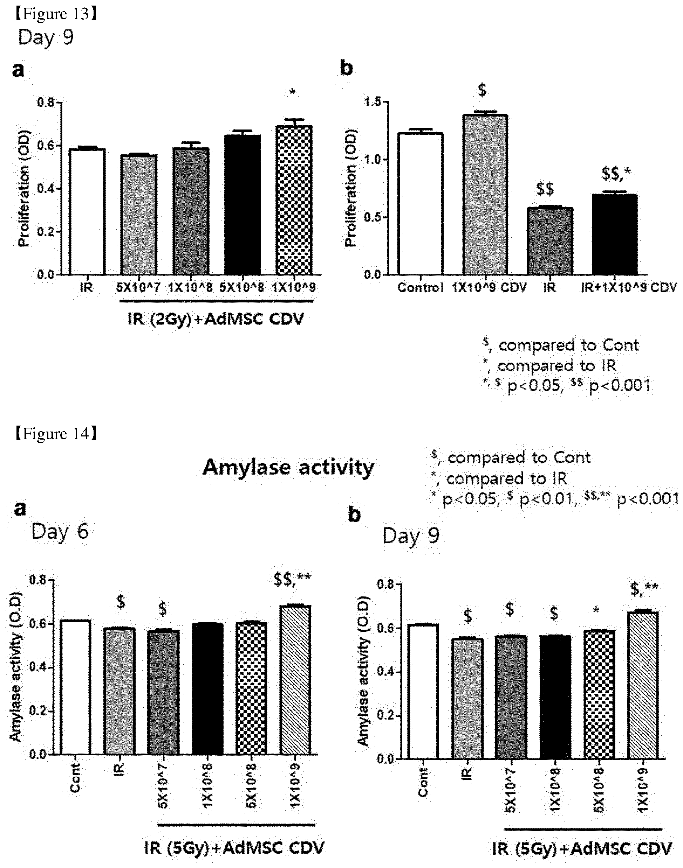

In addition, the proliferation capacity measured 9 days after irradiation damage was classified into the normal control group (no irradiation treatment and no AdMSC-CDV treatment), IR group (no AdMSC-CDV treatment and 2 Gy of irradiation (IR) treatment only), AdMSC-CDV group (no irradiation treatment and 1 × 10⁹ particles/ml of AdMSC-CDV treatment) and IR + AdMSC-CDV group (2 Gy of IR treatment and 1 × 10⁸, 5 × 10⁸, or 1 × 10⁹ particles/ml of AdMSC-CDV treatment), and the comparison results are shown in FIGS. 13A and 13B.

As shown in FIG. 12A, after treatment with AdMSC-CDV at a concentration of 5 × 10⁷ to 1 × 10⁹/ml to sublingual gland cells irradiated with 0 Gy of radiation, cell proliferation capacity was confirmed on 1, 3, 6, 9 and 12th day. As a result, it was confirmed that the cell proliferation capacity was increased in the groups treated with 1 × 10⁹/ml concentration from 6th day. Further, it was confirmed that this effect persisted until the 12th day.

As shown in FIG. 12B, after treatment with AdMSC-CDV at a concentration of 5 × 10⁷ to 1 × 10⁹/ml to sublingual gland cells irradiated with 2 Gy of radiation, cell proliferation capacity was confirmed on days 1, 3, 6, 9 and 12th day. As a result, it was confirmed that the cell proliferation capacity was increased in the groups treated with 1 × 10⁹/ml concentration of AdMSC-CDV from 9th day compared to the group untreated with AdMSC-CDV. Further, it was confirmed that this effect persisted until the 12th day.

As shown in FIG. 12C, after treatment with AdMSC-CDV at a concentration of 5 × 10⁷ to 1 × 10⁹/ml to sublingual gland cells irradiated with 5 Gy of radiation, cell proliferation capacity was confirmed on days 1, 3, 6, 9 and 12th day. As a result, it was confirmed that the cell proliferation capacity was increased in the group treated with 1 × 10⁹/ml concentration of AdMSC-CDV from 9th day compared to the group untreated with AdMSC-CDV. Further, it was confirmed that this effect persisted until the 12th day.

As shown in FIG 13A, the sublingual gland cells irradiated with 2 Gy radiation were treated with AdMSC-CDV by concentration, and the cell proliferation capacity after 9 days was confirmed. It was confirmed that the cell proliferation capacity of the group irradiated with 2 Gy of radiation and treated with AdMSC-CDV at a concentration of 1 × 10⁹/ml was increased compared to the group irradiated with only 2 Gy of radiation.

As shown in FIG. 13B, it was confirmed that the cell proliferation capacity of the group treated with AdMSC-CDV at a concentration of 1 × 10⁹/ml without irradiation was much higher compared to that of the untreated group, and the cell proliferation capacity of the group irradiated with only 2 Gy of radiation was decreased compared to that of the untreated group, but the cell proliferation capacity of the group irradiated with 2 Gy of radiation and treated with AdMSC-CDV at a concentration of 1 × 10⁹/ml was much higher than that of the group irradiated with only 2 Gy of radiation.

Combining the above data, it was confirmed that AdMSC-CDV enhances the proliferation capacity of sublingual gland cells, and the proliferation capacity of sublingual gland cells damaged by irradiation is also increased by AdMSC-CDV.

### 6.3 Confirmation of effect of promoting amylase activity of adipose-derived mesenchymal stem cell-derived vesicles on sublingual gland tissue damaged by irradiation

An experiment was conducted to confirm the amylase activity promoting effect of adipose-derived mesenchymal stem cell-derived vesicles on sublingual gland tissue damaged by irradiation. Specifically, sublingual gland cells prepared in Example 1 were irradiated in the same manner as in Example 2. The group in which sublingual gland cells were not treated and the group irradiated with only 5 Gy radiation were set as controls. The group in which sublingual gland cells were irradiated with 5 Gy radiation and treated with AdMSC-CDV prepared in Example 3 at a concentration of 5 × 10⁷ to 1 × 10⁹/ml was set as the experimental group. Specifically, the amylase activity was measured in the same method as in Example 4.3. The results measured 6 days after irradiation are shown in FIG. 14A, and the results measured 9 days after irradiation are shown in FIG. 14B.

As shown in FIG. 14A, it was confirmed that the amylase activity of the group irradiated with only 5 Gy radiation was reduced compared to the untreated group. It was confirmed that the amylase activity of the group irradiated with 5 Gy radiation and treated with 1 × 10⁹/ml AdMSC-CDV had a level higher than the amylase activity of the untreated group.

As shown in FIG. 14B, it was confirmed that the amylase activity of the group irradiated with only 5 Gy radiation was reduced compared to the untreated group. Further, it was confirmed that the amylase activity of the group irradiated with 5 Gy radiation and treated with 5 × 10⁸/ml AdMSC-CDV was increased compared to those of the group irradiated with only 5 Gy radiation, the group irradiated with 5 Gy radiation and treated with 5 × 10⁷/ml AdMSC-CDV and the group irradiated with 5 Gy radiation and treated with 1 × 10⁸/ml AdMSC-CDV. Further, it was confirmed that the amylase activity of the group irradiated with 5 Gy radiation and treated with 1 × 10⁹/ml AdMSC-CDV had a level higher than the amylase activity of the untreated group.

That is, it was confirmed that AdMSC-CDV promotes the amylase activity of sublingual gland cells, and that the amylase activity of sublingual gland cells damaged by radiation is also restored by AdMSC-CDV.

### 6.4 Confirmation of effect of enhancing transepithelial resistance (TEER) of adipose-derived mesenchymal stem cell-derived vesicles on sublingual gland tissue damaged by irradiation

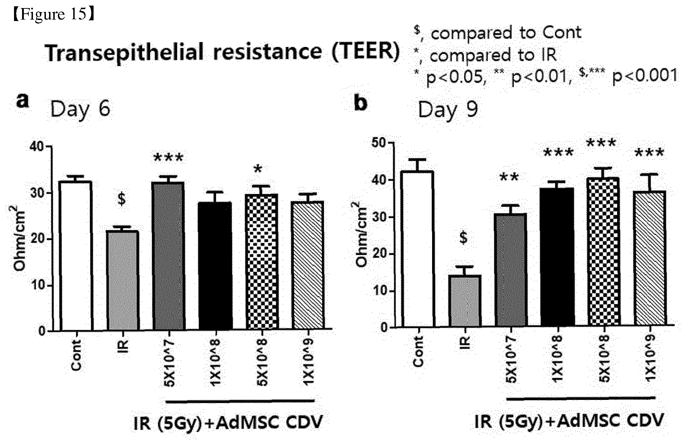

An experiment was conducted to confirm the transepithelial resistance enhancing effect of adipose-derived mesenchymal stem cell-derived vesicles on sublingual gland tissue damaged by radiation. Specifically, sublingual gland cells prepared in Example 1 were irradiated in the same manner as in Example 2. The group in which sublingual gland cells were not treated and the group irradiated with only 5 Gy radiation were set as controls. The group in which sublingual gland cells were irradiated with 5 Gy radiation and treated with AdMSC-CDV prepared in Example 3 at a concentration of 5 × 10⁷ to 1 × 10⁹/ml was set as the experimental group. Transepithelial resistance was measured in the same manner as in Example 4.4, and the measurement results are shown in FIGS. 15A and 15B.

As shown in FIG. 15A, transepithelial resistance (TEER) was measured 6 days after the sublingual gland cells were irradiated. It was confirmed that the transepithelial resistance (TEER) of all groups irradiated with radiation was significantly reduced compared to the untreated group, and the transepithelial resistance (TEER) of groups irradiated with 5 Gy radiation and then treated with 5 × 10⁷ to 5 × 10⁸/ml AdMSC-CDV was higher compared to those of the groups irradiated with only 5 Gy radiation.

As shown in FIG. 15B, the transepithelial resistance (TEER) was measured 9 days after irradiation, and the results indicated that the transepithelial resistance (TEER) of all groups irradiated with 5 Gy radiation was reduced compared to the untreated group, and the transepithelial resistance (TEER) of groups irradiated with 5 Gy radiation and then treated with 5 × 10⁷ to 1 × 10⁹/ml AdMSC-CDV was higher compared to those of the groups irradiated with only 5 Gy radiation.

That is, it was confirmed that AdMSC-CDV increases the transepithelial resistance (TEER) of sublingual gland cells, and the transepithelial resistance (TEER) of sublingual gland cells damaged by irradiation is also restored by AdMSC-CDV.

### Example 7. Confirmation of effect of enhancing proliferation capacity of cell-derived vesicles compared to naturally secreted exosomes

### 7.1 Preparation of adipocyte-derived mesenchymal stem cells (AdMSC)-derived naturally secreted exosomes

Naturally secreted exosomes were obtained from adipocyte-derived mesenchymal stem cell culture medium through ultracentrifuge or tangential flow filtration (TFF). Specifically, the existing culture medium of mesenchymal stem cells cultured in the cell complete growth medium was removed, and then the exosome-depleted culture medium obtained by ultracentrifugation at 120,000 g for 16 hours was added. Then, they were incubated for 24 hours. Thereafter, naturally secreted exosomes were isolated from the culture medium through the following sequential ultracentrifugation. First, the supernatant was obtained by sequential centrifugation for 10 minutes at 300 g, 10 minutes at 2,000 g, and 30 minutes at 10,000 g or the filtrate was obtained by passing through a membrane column through TFF in the same manner as in Example 3. Thereafter, ultracentrifugation was performed at 120,000 g for 2 hours to obtain a precipitated layer. After resuspending this in phosphate-buffered saline (PBS), ultracentrifugation was performed again for 2 or 3 hours at 120,000 g conditions for washing. Thereafter, the precipitated layer was resuspended in phosphate-buffered saline (PBS) to obtain a suspension. The suspension was filtered through a 0.2 um Steritop Threaded Bottle Top Filter (Millipore, USA) to obtain naturally secreted exosomes derived from mesenchymal stem cells.

### 7.2 Confirmation of effect of enhancing proliferation capacity of mesenchymal stem cell-derived vesicles compared to naturally secreted exosomes

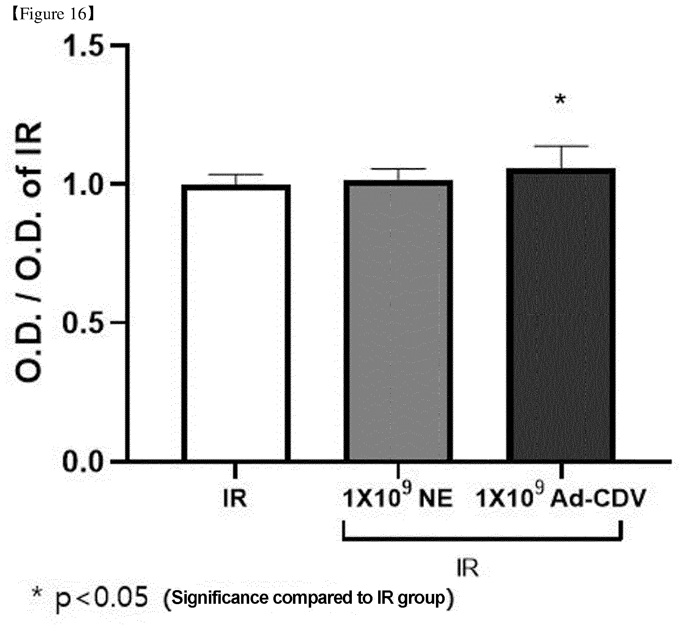

An experiment was conducted to compare the effect of enhancing the proliferation capacity of naturally secreted exosomes and the proliferation capacity of cell-derived vesicles on parotid gland tissue damaged by radiation. Specifically, the parotid gland cells damaged by the 5 Gy radiation prepared in Example 2 were used. They were set as an untreated experimental group (IR), an experimental group (IR + AdMSC NE) treated with 1 × 10⁹ naturally secreted exosomes prepared in Example 7.1, and an experimental group (IR + AdMSC CDV) treated with 1 × 10⁹ AdMSC CDV prepared in Example 3. On the 9th day of exposure to radiation, the proliferation capacity of each experimental group was measured in the same manner as in Example 4.1, and the measurement results are shown in FIG. 16 and Table 1.

**[Table 1]**

| | IR | IR + AdMSC NE | IR + AdMSC CDV |
|---|---|---|---|
| Mean ± standard deviation | 1.00 ± 0.04 | 1.01 ± 0.04 | 1.06 ± 0.08 |

As shown in FIG. 16 and Table 1, in the case of the experimental group (IR + AdMSC NE) treated with naturally secreted exosomes (AdMSC NE), the proliferation capacity of parotid glands damaged by radiation was not restored compared to the untreated experimental group (IR). However, it was confirmed that in the case of the experimental group (IR + AdMSC CDV) treated with AdMSC CDV, the parotid gland proliferation capacity was recovered to a significant extent compared to the untreated experimental group (IR).

### Example 8. Confirmation of effect of enhancing transepithelial resistance (TEER) of cell-derived vesicles compared to naturally secreted exosomes

### 8.1 Confirmation of effect of enhancing transepithelial resistance (TEER) of adipocyte-derived mesenchymal stem cell-derived vesicle (AdMSC CDV) compared to naturally secreted exosomes

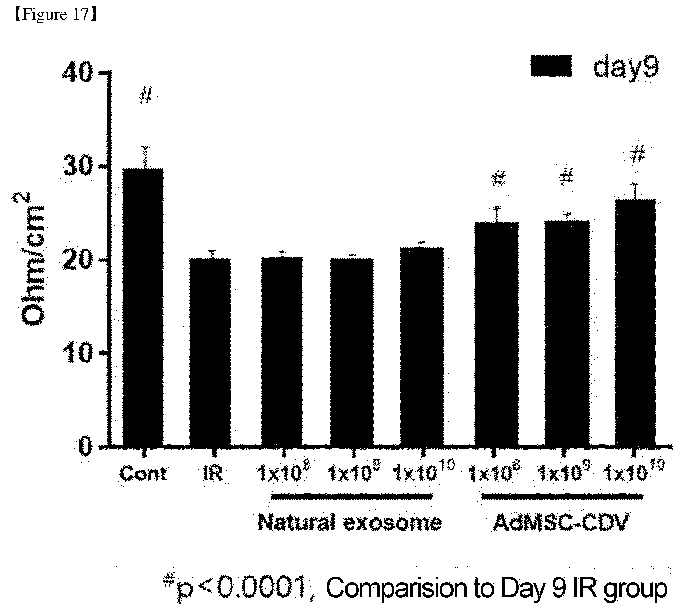

An experiment was conducted to compare the enhancing effect of transepithelial resistance of naturally secreted exosomes and transepithelial resistance of cell-derived vesicles on parotid gland tissue damaged by radiation. Specifically, the experimental groups were set as a control group in which parotid gland cells were not irradiated with radiation (con), an untreated experimental group for parotid gland cells damaged by 5 Gy radiation prepared in Example 2 (IR), an experimental group treated with 1 × 10⁸, 1 × 10⁹, and 1 × 10¹⁰ naturally secreted exosomes prepared in Example 7.1 (AdMSC NE), and an experimental group treated with 1 × 10⁸, 1 × 10⁹, and 1 × 10¹⁰ AdMSC CDV prepared in Example 3 (IR + AdMSC CDV). On the 9th day of exposure to radiation, the transepithelial resistance of each experimental group was measured in the same manner as in Example 4.4, and the measurement results are shown in FIG. 17.

As shown in FIG. 17, it was confirmed that in the experimental group treated with naturally secreted exosomes (AdMSC NE), regardless of the number of exosomes, there was no significant difference from the untreated experimental group (IR). However, it was confirmed that transepithelial resistance in all experimental groups treated with AdMSC CDV was increased compared to the untreated experimental group (IR).

### 8.2 Confirmation of effect of enhancing transepithelial resistance (TEER) of umbilical cord-derived mesenchymal stem cell-derived vesicle (UC-MSC CDV) compared to naturally secreted exosomes

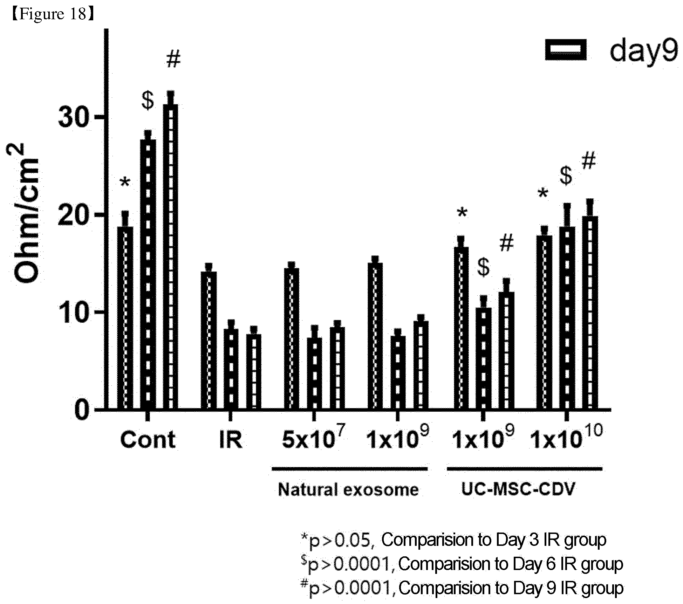

An experiment was performed to determine whether transepithelial resistance increased even when the cell of cell-derived vesicles was changed. Specifically, cell-derived vesicles (UC-MSC CDV) were prepared from umbilical cord-derived mesenchymal stem cells in the same manner as in Example 3, and naturally secreted exosomes (UC-MSC NE) were prepared in the same manner as in Example 7.1. The parotid gland cells damaged by the 5 Gy radiation prepared in Example 2 were used for the experiment. The experimental groups were set as an untreated experimental group (IR), an experimental group treated 5 × 10⁷ and 1 × 10⁹ naturally secreted exosomes (UC-MSC NE), and an experimental group treated 1 × 10⁹ and 1 × 10¹⁰ UC-MSC CDV (UC-MSC CDV). Transepithelial resistance of naturally secreted exosomes isolated from UC-MSC CDV and umbilical cord-derived mesenchymal stem cells in the same manner as in Example 9.1 was measured on the 3rd, 6th, and 9th days of radiation exposure. The measurement results are shown in FIG. 18.

As shown in FIG. 18, it was confirmed that in the experimental group treated with naturally secreted exosomes (UC-MSC NE), regardless of the number of exosomes and the elapsed time of exposure to radiation, there was no significant difference from the untreated experimental group (IR), but all experimental groups treated UC-MSC CDV had increased transepithelial resistance compared to the untreated experimental group (IR). These results indicated that the cell-derived vesicle of the present invention has an effect of enhancing transepithelial resistance when treated with cells, unlike naturally secreted exosomes, even if the derived cells are different.

### Example 9. Confirmation of effect of promoting amylase activity of mesenchymal stem cell-derived vesicles compared to naturally occurring exosomes in animal models

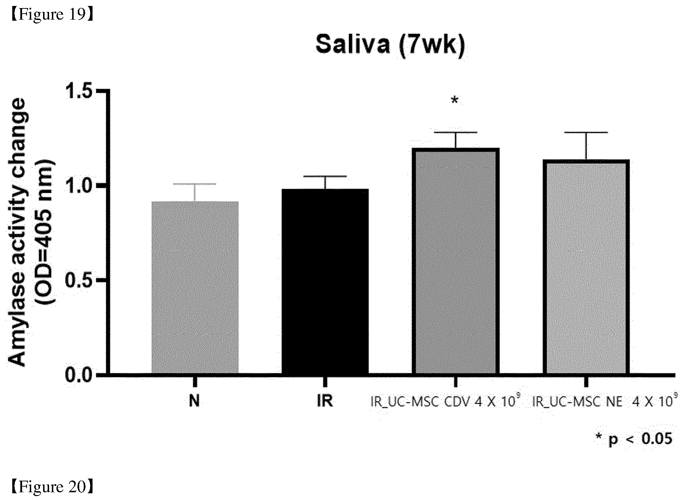

An experiment was conducted to confirm whether it was effective in restoring the function of the salivary gland, when the mesenchymal stem cell-derived vesicle of the present invention was applied to an animal model in which the salivary gland was actually damaged. Experimental mice (4-week-old female C3H; 18-22 g) were purchased from Orient Bio Resource Center (Orient Bio., Seongnam, Korea), and were bred according to the guidelines for laboratory animals at Inha University. Twenty mice were divided into the normal experimental group (Cont), an experimental group irradiated with 15 Gy radiation (IR), an experimental group irradiated with radiation and treated with 4 × 10⁹ UC-MSC-CDV (IR + UC-MSC-CDV), and an experimental group irradiated with radiation and treated with 4 × 10⁹ UC-MSC-NE (IR + UC-MSC-NE). All animal experiments were carried out after obtaining approval from the Institutional Animal Care and Use Committee of Inha University (INHA 1612140464). Saliva obtained from mice of the four experimental groups set above was centrifuged at 1,500 g at 4°C for 10 minutes to obtain only the supernatant, and amylase activity was measured according to the amylase assay protocol (Abcam, USA). 100 uL of the reaction mixture prepared in advance was added in 96 wells containing saliva (50 uL), and then they were mixed well. Then they were reacted at room temperature for 10 minutes and 60 minutes. Then each absorbance was measured at 405 nm. The difference between the absorbance when reacted for 60 minutes and absorbance when reacted for 10 minutes was calculated and converted into amylase activity, and the results are shown in FIG. 19.

As shown in FIG. 19, the mouse model having salivary gland tissue damaged by radiation treated with UC-MSC NE showed no significant change in amylase activity. However, the mouse model treated with UC-MSC CDV showed a significant increase in amylase activity. These results confirmed in the *in vivo* stage that the cell-derived vesicle of the present invention has a therapeutic effect on salivary gland tissue damage.

### Example 10. Confirmation of effect of increasing salivary secretion amount of mesenchymal stem cell-derived vesicles

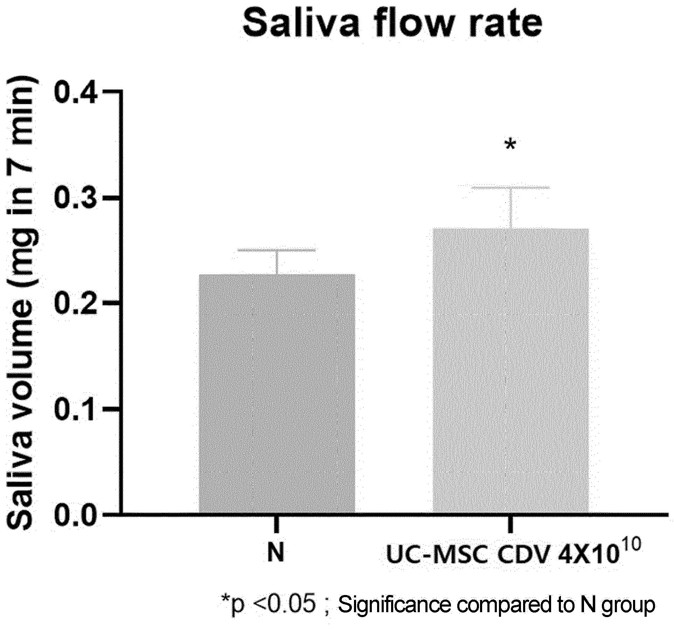

An experiment was conducted to confirm whether it had an effect of increasing salivation amount when the mesenchymal stem cell-derived vesicle of the present invention was applied to a mouse model. Specifically, experimental mice (4-week-old female C3H; 18-22 g) were anesthetized by intraperitoneal injection of ketamine (100 mg/kg), and 10⁹/uL of UC-MSC CDV 40 uL was injected locally to both submandibular glands using an insulin syringe. After injection, it was sutured. Then, 7 weeks later, the mice were anesthetized by injection of ketamine (100 mg/kg, i.p.). The muscarinic cholinergic agonist, pilocarpine (0.2 mg/kg, i.p.) was injected. The saliva was collected from the mouths of the mice for 7 minutes immediately after injection. The collected saliva was weighted, and the amount of saliva secretion was measured. The results are shown in FIG. 20.

As shown in FIG. 20, it was confirmed that the amount of saliva secretion of mice treated with UC-MSC CDV was significantly increased compared to untreated mice as a control group. These results confirmed that the cell-derived vesicle of the present invention has the effect of increasing the amount of salivation.

Hereinafter, a formulation example of a pharmaceutical composition for preventing or treating salivary gland disease, including the cell-derived vesicle of the present invention will be described, but it is not intended to limit the present invention, but merely to describe it in detail.

### Formulation Example 1. Preparation of powder

100 mg of cell-derived vesicle of the present invention
1 g of lactose

The above ingredients were mixed and filled in an airtight cloth to prepare a powder.

### Formulation Example 2. Preparation of tablets

100 mg of cell-derived vesicle of the present invention
100 mg of corn starch
100 mg of lactose
2 mg of magnesium stearate

After mixing the above ingredients, tablets were prepared by tableting according to a conventional manufacturing method of tablets.

### Formulation Example 3. Preparation of capsules

100 mg of cell-derived vesicle of the present invention
100 mg of corn starch
100 mg of lactose
2 mg of magnesium stearate

After mixing the above ingredients, the capsules were prepared by filling in gelatin capsules according to a conventional manufacturing method of capsules.

### Formulation Example 4. Preparation of pills

100 mg of cell-derived vesicle of the present invention
1.5 g of lactose
1 g of glycerin
0.5 g of xylitol

After mixing the above ingredients, it was prepared so as to be 4 g per 1 pill according to a conventional method.

### Formulation Example 5. Preparation of granules

100 mg of cell-derived vesicle of the present invention
50 mg of soybean extract
200 mg of glucose
600 mg of starch

After mixing the above ingredients, 100 mg of 30% ethanol was added, and they were dried at 60°C to form granules, and then the granules were filled in a bag.

### Formulation Example 6. Preparation of injection

10 mg of cell-derived vesicle of the present invention
180 mg of mannitol
2,974 mg of sterile distilled water for injection
26 mg of Na₂HPO₄2H₂O

It was prepared in the content of the above ingredients per 1 ampoule (2 ml) according to a conventional method for preparing injections.

### Formulation Example 7. Tablet-type health functional food

15% by weight of Octacosanol powder, 15% by weight of lactose hydrolyzate powder, 15% by weight of soy protein isolated powder, 15% by weight of chitooligosaccharide, 10% by weight of yeast extract powder, 10% by weight of vitamin and mineral mixture, 4.6% by weight of magnesium stearate, 0.2% by weight of titanium dioxide, 0.2% by weight of glycerin fatty acid ester, and 20% by weight of the cell-derived vesicle of the present invention were blended in a conventional method to prepare a tablet-type health functional food.

### Formulation Example 8. Healthy drink

A healthy drink is prepared by a conventional method by mixing 5% by weight of honey, 3% by weight of fructose, 0.0001% by weight of riboflavin sodium hydrochloride, 0.0001% by weight of pyridoxine hydrochloride, 86.9998% by weight of water and 5% by weight of the cell-derived vesicle of the present invention.

## Claims

1. A pharmaceutical composition for preventing or treating salivary gland disease, the composition comprising cell-derived vesicles prepared by a manufacturing method comprising a step of extruding a sample containing cells into micropores.

2. The pharmaceutical composition of claim 1, wherein the step of extruding the sample containing cells into the micropores is a step of sequentially extruding the sample through a large-sized micropore to a small-sized micropore.

3. The pharmaceutical composition of claim 1, wherein the micropores have a diameter of 0.1 µm to 20 µm.

4. The pharmaceutical composition of any one of claims 1 to 3, wherein the cell includes at least one selected from the group consisting of mesenchymal stem cells, induced pluripotent stem cells, embryonic stem cells, salivary gland stem cells, acinar cells, myoepithelial cells, and platelets.

5. The pharmaceutical composition of any one of claims 1 to 3, wherein the salivary gland disease includes any one selected from the group consisting of irradiation and radioactive isotope damage to the salivary gland, dry mouth, infectious acute sialadenitis, infectious chronic sialadenitis, tuberculosis, Sjogren's syndrome, sialolithiasis, sialadenosis, salivary gland tumor, and salivary gland dysfunction due to aging.

6. The pharmaceutical composition of any one of claims 1 to 3, wherein the cell-derived vesicle is treated to salivary gland tissue to enhance cell proliferation capacity of salivary gland, amylase activity secreted by the salivary glands, or transepithelial resistance (TEER) of salivary gland cells compared to natural secreted exosomes and to increase an amount of salivary secretion.

7. The pharmaceutical composition of any one of claims 1 to 3, wherein the salivary gland is any one selected from the group consisting of submandibular gland, sublingual gland and parotid gland.

8. A health functional food composition for preventing or alleviating salivary gland disease, the composition comprising cell-derived vesicles prepared by a manufacturing method comprising a step of extruding a sample containing cells into micropores.

9. A method for preventing or treating salivary gland disease, the method comprising a step of treating a subject with cell-derived vesicles prepared by a manufacturing method comprising a step of extruding a sample containing cells into micropores.

10. Use of cell-derived vesicles as a medicament for preventing or treating salivary gland disease, wherein the cell-derived vesicles prepared by a manufacturing method comprising a step of extruding a sample containing cells into micropores.

11. Use of cell-derived vesicles prepared by a manufacturing method comprising a step of extruding a sample containing cells into micropores for manufacturing a pharmaceutical composition for preventing or treating salivary gland disease.
